# EUROPEAN PATENT APPLICATION

(11) **EP 4 353 296 A2**
(43) Date of publication of application: **17.04.2024**
(21) Application number: 23211171.6
(22) Date of filing: 15.05.2018
(51) Int. Cl.: A61M 39/02

(54) **TRANS-SEPTAL CLOSURE DEVICE**

(30) Priority: 16.05.2017 US 201762507037 P; 10.05.2018 US 201815975977
(62) Divisional of application: 18803099.3
(71) Applicant: Edwards Lifesciences Corporation, Irvine, CA 92614 (US)
(72) Inventor: BAK-BOYCHUCK, Gregory, IRVINE, CA, 92614 (US); ROWE, Stanton J., IRVINE, CA, 92614 (US); VREEKE, Mark Simon, IRVINE, CA, 92614 (US); VALENCIA, Juan, IRVINE, CA, 92614 (US); SCHOLZ, Tamera Lee, IRVINE, CA, 92614 (US); HENDSBEE, Carey Philip, IRVINE, CA, 92614 (US)
(74) Representative: Eisenführ Speiser

(57) **Abstract**

A septal closure device can include an expandable frame having a central portion defining a lumen. The frame can have a plurality of arms extending radially outward from the central portion. The arms can be connected to the central portion at angularly spaced locations on the central portion that intersect a common plane perpendicular to a central axis of the lumen. The closure device can further include a blood occluding member supported on the frame and positioned to block at least the flow of blood from the left atrium to the right atrium through the lumen of the frame when the device is implanted in the septum.

## Description

### FIELD

The present disclosure relates generally to a method and device for closing a septal defect, or opening in a septum. In particular, the present disclosure relates to a method and device for closing a septal defect, for example a defect in an atrial septum, such that the septal defect can be accessed for reentry through the defect.

### BACKGROUND

A septum may include a thin wall dividing a cavity into two smaller structures. An atrial septum is a wall of tissue separating the left and right atria of the heart. A ventricular septum is a wall of tissue separating the left and right ventricles of the heart. A septal defect may include a perforation or hole passing through the septum. A septal defect can occur congenitally or by puncturing the septum with a medical device to access a location within the heart.

The atrial septum may be viewed like the femoral artery in years to come. The femoral artery is an access point for many catheterization laboratory procedures, with a smaller percentage of procedures utilizing venous or radial artery access. Likewise, the atrial septum is a point of percutaneous access for atrial fibrillation therapy, left atrial appendage closure, percutaneous mitral valve reset, and percutaneous mitral valve replacement. In these and other procedures, devices may traverse across the atrial septum and, by doing so, may leave a defect or orifice in the atrial septum that cannot close spontaneously. Currently, these defects are closed using devices, such as plugs, that may close the defect but do not allow for re-access through the septum. Thus a need exists for improved closure devices for closing a septal defect and for re-accessing the left side of the heart in subsequent procedures.

### SUMMARY

In one representative embodiment, a closure device for implantation in an orifice formed in an organ of a patient's body comprises an expandable frame comprising a central portion defining a lumen, the lumen defining a central axis. The frame can further comprise a plurality of arms extending radially outward from the central portion, the frame being configured to expand and contract between a compressed configuration for delivery through the patient's vasculature and an expanded configuration in which the arms extend radially outwardly from the central portion. All of the arms can be connected to the central portion at angularly spaced locations on the central portion that intersect a common plane perpendicular to the central axis. The closure device can further comprise a blood occluding member supported on the frame and positioned to block at least the flow of blood from one side of the orifice to the other side of the orifice through the lumen of the frame.

In some embodiments, the plurality of arms comprises a first set of two or more arms that can be positioned on one side of the orifice and a second set of two or more arms that can be positioned on the other side of the orifice.

In some embodiments, there are a total of four arms, including exactly two arms in the first set and exactly two arms in the second set.

In some embodiments, the arms of the first set extend from opposing sides of the central portion and the arms of the second set extend from opposing sides of the central portion.

In some embodiments, the arms do not overlap with each other when in the expanded configuration.

In some embodiments, each of the arms has a first portion where it is connected to the central portion and a relatively wider, second portion spaced from the central portion.

In some embodiments, the occluding member comprises a bioresorbable material.

In some embodiments, the occluding member comprises an electro-spun polymer.

In some embodiments, the occluding member comprises a fabric.

In some embodiments, the occluding member comprises a foam.

In some embodiments, the central portion is further expandable from the expanded configuration when a medical instrument is inserted through the lumen.

In some embodiments, the arms are coplanar with the central portion when the frame is in the expanded configuration.

In some embodiments, the central portion of the frame comprises a circumference and each arm is connected to the central portion at spaced apart locations on the circumference.

In some embodiments, the central portion of the frame comprises a central loop and each arm comprises a respective loop connected to the central loop at spaced apart locations around the central loop.

In some embodiments, the blood occluding member is configured to be punctured by a medical instrument.

In another representative embodiment, a method of making an implantable closure device comprises cutting a frame from a flat piece of metal, the frame having a central portion defining a lumen and a plurality of arms extending radially outward from the central portion, one or more of the arms being configured to be positioned against tissue on one side of an orifice in a patient's body and one or more of the arms being configured to be positioned against tissue on the opposite side of the orifice. The method can further comprise securing a blood-occluding member to the frame so as to at least partially cover the lumen.

In some embodiments, the act of cutting comprises laser cutting the frame from the flat piece of metal.

In another representative embodiment, a method of implanting a septal closure device in the atrial septum of a patient's heart comprises inserting a delivery apparatus into the vasculature of the patient. The delivery apparatus can comprise a sheath containing a septal closure device in a compressed configuration, the closure device comprising a frame including a central portion and a plurality of arms attached to the central portion at spaced apart locations along a circumference of the central portion. The method further comprises advancing at least a distal end portion of the sheath across the atrial septum of the patient's heart and deploying the closure device from the sheath such that one or more first arms of the plurality of arms contact the septum in the left atrium and one or more second arms of the plurality of arms contact the septum in the right atrium. The closure device can further comprises a blood occluding member supported on the frame that blocks at least the flow of blood from the left to the right atrium through the central portion of the frame.

In some embodiments, the method further comprises, after deploying the closure device, inserting a medical instrument through the blood occluding member and performing a medical procedure in the left side of the heart using the medical instrument.

In some embodiments, deploying the closure device further comprises deploying the one or more first arms from the sheath to allow the one or more first arms to expand in the left atrium while the one or more second arms remain connected to a shaft of the delivery apparatus, rotating the shaft to rotate the closure device, and releasing the one or more second arms from the shaft, allowing the one or more second arms to expand in the right atrium.

In some embodiments, the method further comprises positioning a distal end portion of the delivery apparatus at an acute angle relative to the septum while deploying the closure device from the sheath.

In some embodiments, deploying the closure device further comprises pivoting the closure device relative to the delivery apparatus while the closure device remains connected to the delivery apparatus.

In some embodiments, the delivery apparatus includes sutures releasably attached to the one or more second arms and the method further comprises removing the sutures from the one or more second arms after deploying the closure device.

In some embodiments, the one or more first arms comprises exactly two arms and the one or more second arms comprises exactly two arms.

In another representative embodiment, a shunt, such as for promoting blood flow from the left atrium into the right atrium, can comprise any of the frames described above without a blood occluding member.

In another representative embodiment, a septal closure device for implantation in the atrial septum of a patient's heart comprises an expandable foam body. The foam body can comprise first and second opposing end portions. The body can be configured to expand and contract between a compressed configuration for delivery through the patient's vasculature and an expanded configuration in which the first and second end portions are positioned on opposing sides of the atrial septum.

In some embodiments, the body further comprises a central portion and the first and second end portions extend radially outwardly from opposing ends of the central portion when the body is in the expanded configuration.

In some embodiments, the closure device further comprises a radiopaque additive within the foam body.

In some embodiments, the foam body comprises a bioresorbable material.

In another representative embodiment, a method of implanting a closure device in an orifice formed in an organ of a patient's body is provided. The method can comprise inserting a delivery apparatus into the vasculature of the patient, the delivery apparatus comprising a sheath containing a closure device in a compressed configuration, the closure device comprising a frame including a central portion and a plurality of arms attached to the central portion at spaced apart locations along a circumference of the central portion; advancing at least a distal end portion of the sheath through the orifice; and deploying the closure device from the sheath such that one or more first arms of the plurality of arms contact tissue on a first side of the orifice and one or more second arms of the plurality of arms contact tissue on an opposing, second side of the orifice, wherein the closure device further comprises a blood occluding member supported on the frame to block at least the flow of blood from the first side to the second side through the central portion.

In another representative embodiment, an implantable medical device for implantation in an orifice formed in an organ of a patient's body comprises a metal frame. The metal frame comprises a plurality of loop shaped anchoring arms extending radially outwardly from a central axis of the frame and a plurality of connecting portions extending between and connecting adjacent anchoring arms. The anchoring arms are angularly arrayed around the central axis and each anchoring arm comprises two radial inner ends with each radial inner end being connected to an adjacent radial inner end of an adjacent anchoring arm by one of the connecting portions. The plurality of anchoring arms comprises a first set of anchoring arms that can be positioned on one side of the orifice and a second set of anchoring arms that can be positioned on the other side of the orifice. The connecting portions define a central lumen of the frame having a first diameter. The frame is configured such that when a medical instrument having a second diameter, greater than the first diameter, is inserted into the central lumen, the connecting portions are pushed radially outwardly to enlarge the lumen under the force of the medical instrument.

In some embodiments, the implantable medical device further comprises a blood occluding member supported on the frame and positioned to block at least the flow of blood from one side of the orifice to the other side of the orifice through the lumen of the frame.

In some embodiments, the plurality of anchoring arms and the connecting portions are formed from a single wire member.

In some embodiments, the plurality of anchoring arms are substantially flat and co-planar with each other.

In some embodiments, each of the plurality of anchoring arms curves away from and back toward an adjacent anchoring arm moving in a radial outward direction along the length of the anchoring arm.

The foregoing and other objects, features, and advantages of the present disclosure will become more apparent from the following detailed description, which proceeds with reference to the accompanying figures.

### BRIEF DESCRIPTION OF THE DRAWINGS

FIG. 1 is a perspective view of a septal closure device, according to one embodiment.
FIG. 2 is a top view of a closure device showing a frame separate from a blood occluding member.
FIG. 3 is a side perspective view of the frame of the closure device of FIG. 1 shown being deployed from a delivery apparatus.
FIGS. 4-6 are side views of the distal end portion of a delivery apparatus shown at various stages of an implantation procedure for implanting the closure device of FIG. 2 in the atrial septum, according to one embodiment.
FIG. 7 is an exploded, perspective view of a closure device showing a frame separate from a blood occluding member, according to another embodiment.
FIG. 8 is a perspective view of the septal closure device of FIG. 7.
FIG. 9 is a top view of an example of a blood occluding member that can be incorporated in a septal closure device, such as the closure device of FIG. 1.
FIG. 10 is a plan view of a septal closure device, according to another embodiment.
FIG. 11 is a plan view of the frame of the closure device of FIG. 10.
FIG. 12 is a perspective view of a septal closure device, according to another embodiment.
FIGS. 13-16 are perspective views of alternative embodiments of frames for a septal closure device.
FIG. 17 is a perspective view of another embodiment of a septal closure device.
FIG. 18 is an exploded perspective view of the closure device of FIG. 17.
FIGS. 19-20 are side views of a septal closure device implanted in an atrial septum at different points in time after implantation.
FIG. 21 is a perspective view of a distal end portion of a delivery apparatus and a septal closure device in a partially expanded position shown being deployed from the delivery apparatus, according to another embodiment.
FIG. 22 is an enlarged, perspective view showing a releasable connection between the closure device and the delivery apparatus of FIG. 21.
FIG. 23 is a perspective view similar to FIG. 21, showing the closure device fully expanded but still connected to the delivery apparatus.
FIG. 24 is an enlarged perspective view showing the closure device of FIGS. 21-23 being released from the delivery apparatus.
FIG. 25 is a side view of a septal closure device, according to another embodiment.
FIG. 26 is a top perspective view of the septal closure device of FIG. 25.
FIGS. 27-30 are side views of the distal end portion of a delivery apparatus shown at various stages of an implantation procedure for implanting the closure device of FIG. 25 in the atrial septum, according to one embodiment.
FIGS. 31A, 31B, 32 and 33 are various views of another embodiment of a septal closure device shown implanted within an orifice of a septum.

### DETAILED DESCRIPTION

In certain embodiments, a septal closure device is suitable to close or reset a septal orifice and allow for re-entry through a septum at the same septal orifice location at a later time as other therapeutic interventions are warranted. In certain embodiments, the closure device that is suitable to provide an access port for accessing the left side of the heart with a catheter or other medical device. As used herein, the term "septal orifice" or "orifice" is used to describe an orifice created by puncturing the septum with a catheter or other medical device and an orifice that occurs congenitally, such as an atrial septal defect (ASD) or a patent foramen ovale (PFO).

The embodiments of the closure device described below are described in the context of occluding or closing an orifice in the atrial septum. The disclosed embodiments also can be implanted in orifices formed in a ventricular septum, the apex or other sections of the heart, or in orifices (surgically or congenitally formed orifices) formed in other organs of the body.

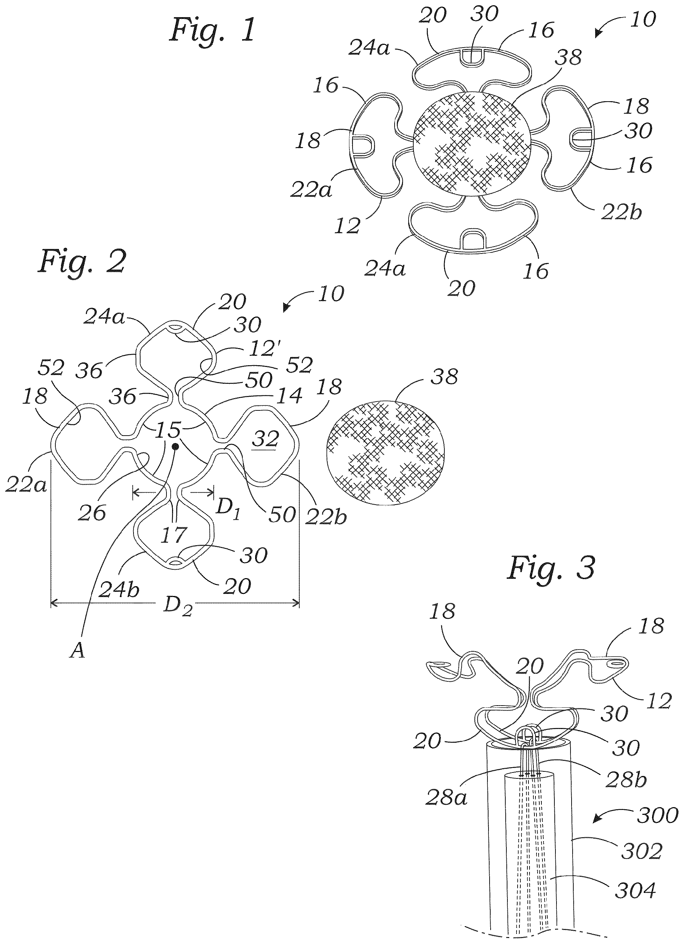

As shown in FIG. 1, a septal closure device 10 can include a frame 12 configured to support a blood occluding member 38. FIG. 2 shows a similar septal closure device 10 having a frame 12' supporting a blood occluding member 38. The frame 12' has a slightly different shape than the frame 12. For ease of description, common components of frames 12, 12' are given the same reference numeral, and except as noted below, references to frame 12 are meant to include frame 12 and frame 12'.

The frame 12 in the illustrated configuration can generally comprise a generally planar body comprising a central portion 14 and a plurality of anchoring arms 16 extending radially outward from the central portion 14. For example, at least four arms can extend from the central portion 14, as shown in the illustrated embodiment, although the frame can have greater than four arms 16 or less than three arms 16 in other embodiments.

The four arms 16 may include a first set of opposing arms 18 (which can also be referred to as the "distal arms" in some embodiments), and a second set of opposing arms 20 (which can also be referred to as the "proximal arms" in some embodiments), extending from the central portion 14. The closure device desirably (although not necessarily) has the same number of arms in the first and second sets so that the clamping force exerted by the arms is evenly distributed against the septum when the device is implanted. In the illustrated embodiment, for example, the first set of arms 18 includes exactly two arms 22a and 22b extending from opposing sides of the central portion 14, and the second set of arms 20 includes exactly two arms 24a and 24b extending from opposing sides of the central portion 14. In other embodiments, the first or second set of arms can include just one arm or more than three arms.

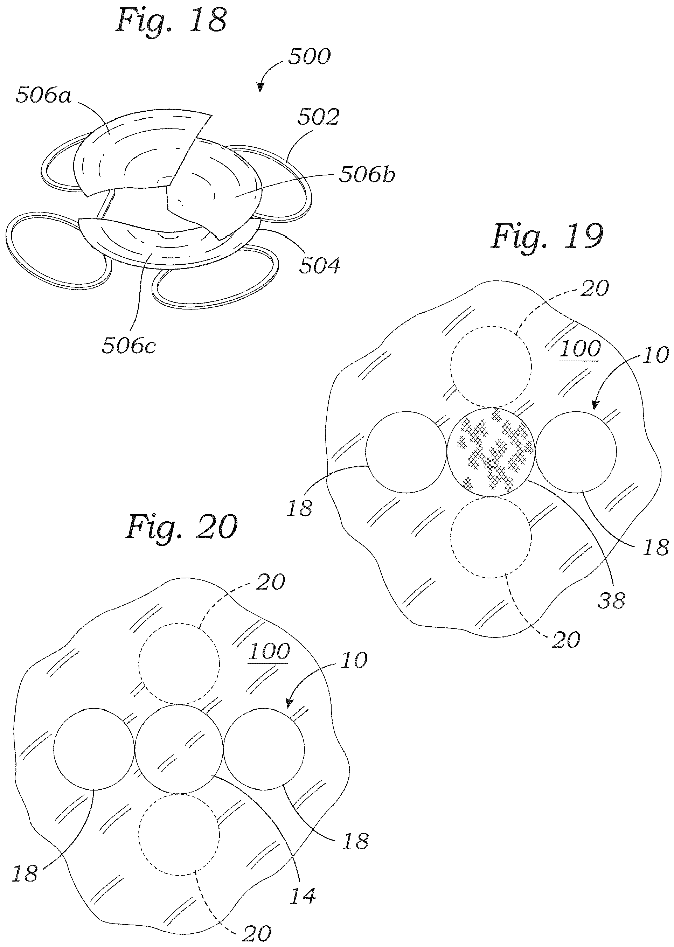

In a deployed or expanded configuration, the arms 16 can extend radially outwardly from the central portion 14. The arms 16 can extend perpendicularly or substantially perpendicularly to a central axis A of the device 10 (the central axis extending through a lumen 26 of the central portion 14 perpendicular to the plane of the page) such that an atrial septum 100 can be compressed or pinched between the first set of arms 18 and the second set of arms 20 when the device 10 is implanted in the atrial septum 100. In other words, when the device 10 is implanted, the first set of arms 18 can be on one side of the atrial septum 100, the second set of arms 20 can be on the other side of the atrial septum 100 and the central portion 14 can be disposed within an orifice or defect 102 (FIGS. 19 and 20) or offset to one side of the septum.

The frame 12 in the illustrated embodiment has a relatively thin and flat profile to avoid or minimize thrombus. Thus, to such ends, the arms 16 can be attached to the central portion 14 at angularly spaced apart locations on the central portion, with the attachment locations intersecting a common plane perpendicular to the central axis; in other words, all of the arms 16 in the illustrated embodiment can be attached to the central portion along the same circumferential path defined by the central portion 14. The illustrated device has a much thinner profile compared to known devices, which typically have anchors at opposite axial ends of the device.

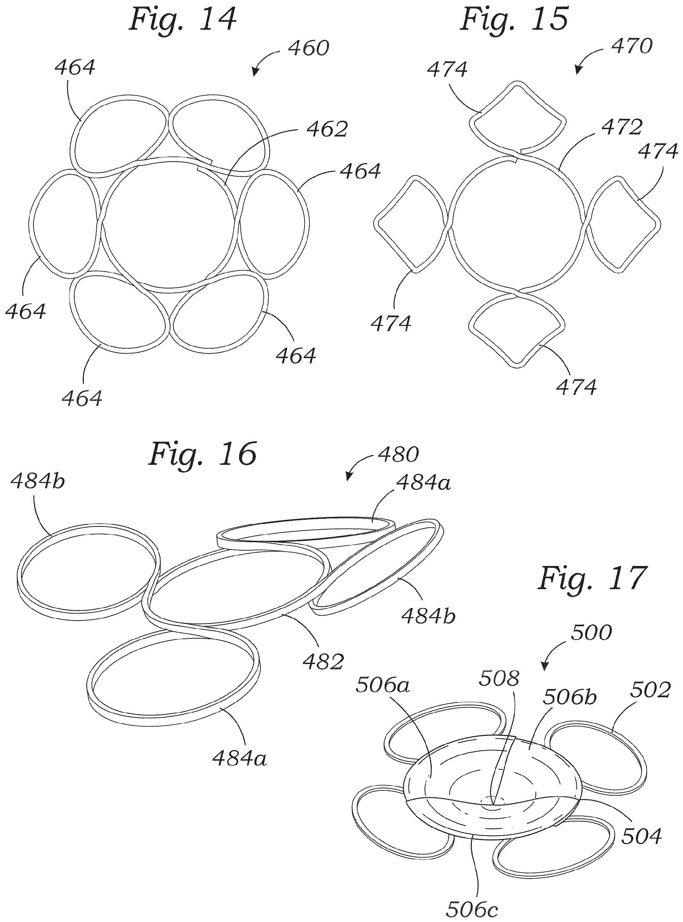

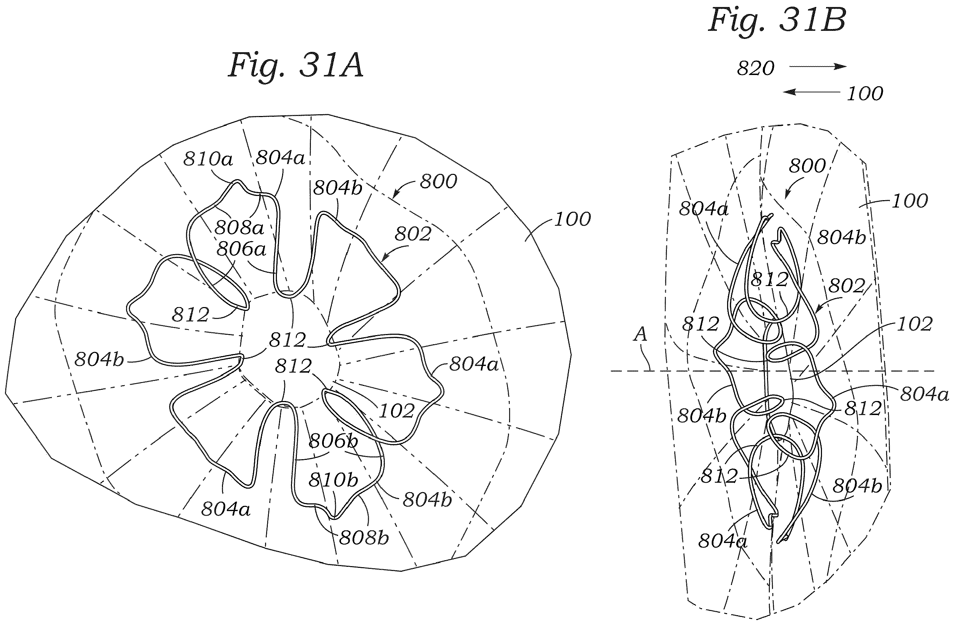

In certain embodiments, the arms 16 and the central portion 14 can be coplanar with each other when the device 10 is in its fully expanded, non-deflected shape; that is, the arms 16 do not have any portions that extend axially away from the central portion 14. It should be understood that once implanted, the first set of arms 18 and the second set of arms 20 may be bent slightly axially away from each other by virtue of the thickness of the septum 100 and may no longer be coplanar. Nonetheless, the device 10 in certain embodiments can be said to have a flat profile with arms that are coplanar with each other and the central when the device is in a non-deflected state. In other embodiments, however, the arms or portions thereof can be heat-set or otherwise shaped to extend axially away from each other or the central portion in a non-deflected state (e.g., frame 480 of FIG. 16 or frame 802 of FIGS. 31A-31B, described below).

Further, in some embodiments, the arms 16 do not overlap with each other in the direction of the central axis when in the expanded configuration; that is, a line parallel to the central axis of the frame does not intersect or extend through more than one arm 16. In particular embodiments, the arms 16 desirably are circumferentially spaced from each other as shown so that there is a gap between adjacent sides of adjacent arms 16.

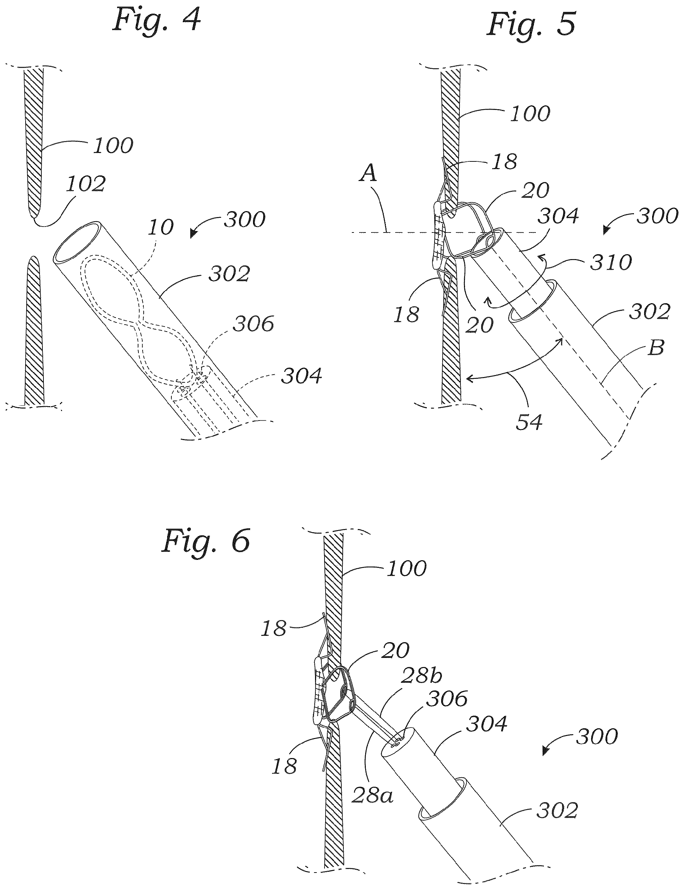

The frame 12 can be radially compressed or constricted to a delivery configuration for delivery to the heart on a delivery apparatus. As shown in FIG. 4, in the delivery configuration, the frame 12 can be placed and retained in a generally compressed configuration in which the first set of arms 18 are folded towards each other along the central axis of the device 10 and the second set of arms 20 are folded towards each other along the central axis of the device 10, such that the first and second set of arms 18, 20, respectively, extend axially and parallel to each other and the central portion 14. When placed in the delivery configuration, the frame 12 can also be radially compressed relative to the deployed configuration.

The frame 12 can include an eyelet 30 disposed at a distal end of each arm (see FIG. 1) for attaching the closure device 10 to a delivery apparatus via one or more attachment structures (e.g., sutures), as further described below. Alternatively, as shown in FIG. 2, the frame 12' can include eyelets 30 disposed at a distal end of two arms, for example the second set of arms 20. An eyelet can project towards the central portion 14, as shown in FIGS. 1 and 2, or it can project away from the central portion 14.

The frame 12 can be self-expandable and can be formed from a shape-memory material, such as Nitinol, so that the frame 12 self-expands from the delivery configuration to the deployed configuration when released or deployed from a delivery apparatus. In alternative embodiments, the frame 12 can be formed from a plastically-expandable material, such as stainless steel or cobalt-chromium alloy, and can be configured to be plastically expanded from the delivery configuration to the deployed configuration by an expansion device, such as an inflatable balloon. The frame 12 can be laser cut or otherwise formed from a flat sheet of metal, such as Nitinol. Alternatively, the frame 12 can be formed by bending one or more metal wires into the form shown.

As shown in FIGS. 1 and 2, the central portion 14 can comprise a central loop shaped member and each of the arms can comprise a respective loop shaped member spaced around the circumference of the central loop shaped member. Additionally and/or alternatively, the central portion lumen 26 can be open at the locations where the central portion 14 branches off into each of the plurality of arms, forming gaps 50. Additionally and/or alternatively, each of the plurality of arms can include an open area 52, each of which can be in communication with the central lumen 26 via a gap 50. In this manner, the central portion 14 forms discrete connecting portions 15 extending between and interconnecting adjacent arms 16. Each arm 16 includes two circumferentially spaced radial inner ends 17 that are connected to adjacent radial inner ends 17 of adjacent arms by respective connecting portions 15. Additionally, as shown in FIG. 2, the arms 16 and the connecting portions 15 of the illustrated frame 12 collectively form a simple closed loop structure wherein a single continuous frame member forms each of the arms and the connecting portions.

Each of the arms 16 can have a variety of shapes that can have a narrow portion 34, at the intersection with the central portion 14, and a wide portion 36, for example at a middle portion of the arm. Some embodiments of the plurality of arms 16 may include a mushroom shape, as shown in FIG. 1, or a diamond shape, as shown in FIG. 2. Alternatively, the arms can circular in shape. Alternatively, the configuration of one or more of the arms 16 in a plurality of arms of a septal closure device 10 can be different from other arms in the plurality of arms 16. In still other embodiments, the arms 16 need not comprise loop shaped members with central openings 52 and instead can comprise elongated wires or strut members that are secured to the central portion at only one end of the wire or strut member.

As shown in FIG. 1, the frame 12 in the deployed configuration can include an inner diameter D 1 and an outer diameter D2. The inner diameter D 1 can be slightly less to slightly greater than that the diameter of the orifice 102 in the septum 100 (see FIG. 4). The outer diameter D2 can be defined by the circumference formed from the radial outermost ends of the arms. The number of arms, the length of the arms, and the inner and outer diameter of the frame 12 can be varied as needed for particular applications of the frame 12.

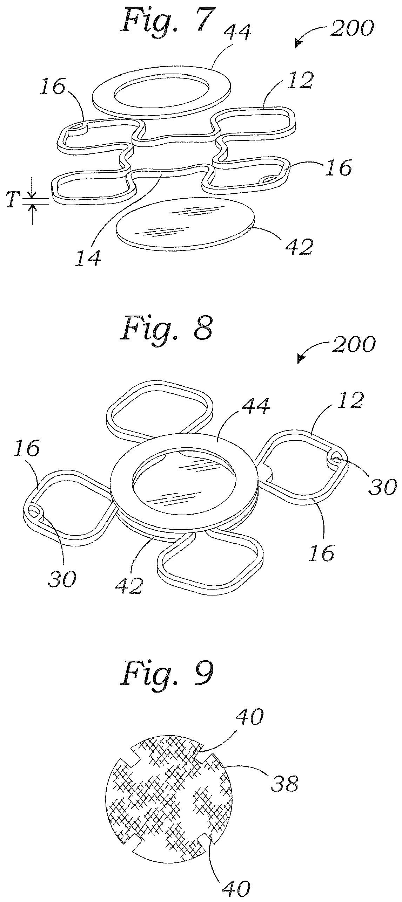

In certain embodiments, the inner diameter D1 can be between about 5 mm and 16 mm, and more specifically, between about 6 mm and 12 mm, with 8 mm being a specific example. The outer diameter D2 can be between about 15 mm and 25 mm, and more specifically, between about 22 mm and 18 mm, with 20 mm being a specific example. The thickness T of the frame 12 (FIG. 7) can be between about 0.1 mm and 0.3 mm, and more specifically, between about 0.15 mm and 0.25 mm, with 0.20 mm being a specific example. The specific dimensions can be varied as needed depending on the size of the orifice in which the implant is to be implanted. For example, for an orifice 102 measuring 4 to 7 mm, D1 can be 8 mm or greater. For an office 102 measuring 8 to 11 mm, D1 can be 12 mm or greater. For an orifice 102 measuring 12 to 15 mm, D1 can be 16 mm or greater. In some embodiments, a manufacturer can provide the implant in three different sizes, which are sufficient for implantation in a range of orifices, for example, orifices measuring 4 to 15 mm.

In one specific implementation, the device 10 has a total surface area of 166 mm², a weight of 0.05 gram, and provides a retention force of at least 3.4 N. In comparison, the Amplatzer septal occluder model 9-PFO-25 (available from St. Jude Medical) has a surface area of 1,389 mm², weighs 0.41 gram, and provides a retention force of 3.6 N. As can be appreciated, the device 10 provides a comparable retention force but uses substantially less metal and therefore is much less susceptible to thrombus formation.

In particular embodiments, the occluding member 38 can be configured to block the flow of blood between the right and left atriums through the closure device 10 and optionally can permit passage of a medical device through the lumen of the closure device 10. For an adult, the normal range of right atrial pressure (RAP) is about 2-6 mmHg and the normal range of left atrial pressure (LAP) is about 4-12 mmHg. Thus, throughout most of the cardiac cycle, the LAP is greater than the RAP. In some embodiments, the occluding member 38 can be configured to block at least the flow of blood from left atrium to the right atrium. In other embodiments, the occluding member 28 can be configured to block the flow of blood between the right and left atriums in both directions throughout the cardiac cycle.

In particular embodiments, the occluding member 38 can comprise one or more sheets or pieces of material that at least partially block or impede the flow of blood through the frame 12. For example, the occluding member 38 can comprise one or more pieces of bioresorbable material, film or cloth that are configured to encourage tissue ingrowth and can degrade over time, leaving just regrown tissue within the central portion 14. For example, the occluding member 38 can comprise one or more pieces of bioresorable electro-spun polymeric material, such as polylactide (PLA), polylactide glycolides (PLGA), polycaprolactone (PLC), polyacrylonitrile (PAN), poly(lactide-co-caprolactone) (PLCL), polygyconate, and polypeptides. Compared to woven fabrics, electro-spun polymers promote faster tissue ingrowth, have faster biodegradation times, are potentially less thrombogenic, and can be created weaker and therefore can be easily punctured with a medical instrument during subsequent recrossing of the closure device.

In other embodiments, the occluding member 38 can comprise one or more sheets of pieces of non-bioresorbable material, such as any of various synthetic fabrics (e.g., polyethylene terephthalate (PET)) or natural tissue (e.g., pericardium). In some embodiments, the occluding member 38 can be completely or substantially impermeable to blood. In other embodiments, the occluding member 38 can be semiporous to blood flow (e.g., a porous fabric). The porous material can be selected to remain porous or to close up and become impermeable or non-porous to blood over time. In a specific implementation, the occluding member can be made of a bio-spun polyurethane having a fiber size between .05 to 1.5 microns and a porosity of between 50% and 80%. The thickness of the occluding member can be between 100 to 200 microns. In another implementation, the occluding member can be made of a bio-spun polymer blend comprising polyurethane and PET, such as a 70/30% blend of polyurethane/PET, having similar fiber sizes and porosity.

In still alternative embodiments, the occluding member 38 can be made of a biocompatible foam, such as polyurethane, PET, silicone, or polyethylene foam.

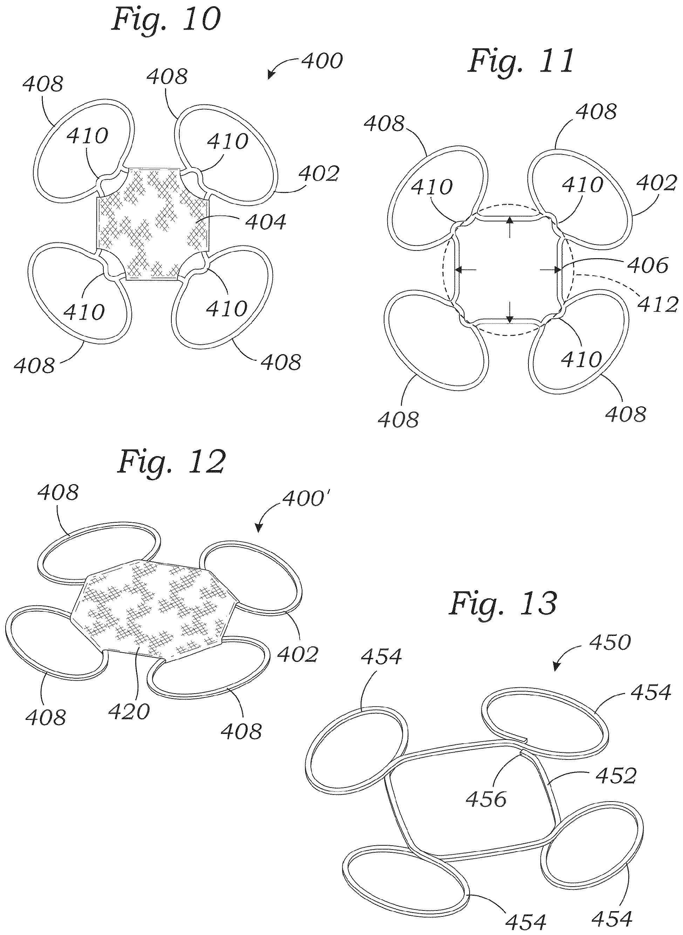

The occluding member 38 can, but need not create a fluid-tight seal with the adjacent tissue of the septum, and instead can, at least initially, permit a small amount of blood flow between the atria (referred to as residual shunting). Over time, the occluding member 38 can promote tissue ingrowth and completely close the orifice 102 and prevent residual shunting between the atria. The occluding member 38 can completely cover the lumen of the central portion 14, as shown in FIG. 1, or the occluding member 38 can cover a portion of the lumen of the central portion 14, as shown in FIG. 10. As discussed in further detail below, the occluding member 38 can be configured such that the septal defect 102 can be accessed for reentry through the defect either before or after occluding member 38 degradation.

The occluding member 38 can be attached to the frame 12 via heat staking, sutures, molding, bonding, weaving and other means known to those skill in the art with the benefit of the present disclosure. For example, the outer edges of the occluding member 38 can be folded over the central portion 14 and then welded to a more central area of the occluding member 38 to fix the occluding member 38 to the frame 12. The occluding member 38 may extend beyond the periphery of the central portion 14, for example up to 2 mm. In some embodiments, the occluding member 38 may have a generally circular shape prior to attachment to the frame 12, as shown in FIG. 2.

Alternatively, as shown in FIG. 9, the occluding member 38 can include a plurality of notches 40 that align with the plurality of arms 16 to aid in the folding of the occluding member 38 over the periphery of the central portion 14. Additionally and/or alternatively, the occluding member 38 may have any shape configured to cover all or a portion of the lumen of a central portion, as known to those skilled in the art with the benefit of the present disclosure.

FIGS. 3-6 illustrate one example of delivering and implanting a septal closure device 10 using an exemplary delivery apparatus 300. While the description proceeds with reference to the closure device 10, the disclosed method and delivery apparatus can be used to implant any of the closure devices described herein. The delivery apparatus 300 can include an outer sheath 302 and an inner shaft 304 (which can also be referred to as a "pusher member" in some embodiments) extending co-axially through the outer sheath 302. The second set of arms 20 of the frame 12 can be releasably connected to the inner shaft 304 via sutures 28 or other releasable retaining structures or mechanisms known to those skilled in the art with the benefit of the present disclosure. The proximal ends of the sheath 302 and the inner shaft 304 can be coupled to a handle (not shown) having appropriate actuators (e.g., knobs) to effect relative longitudinal and/or rotational movement of the outer sheath 302 and the inner shaft 304 and actuators to effect relative movement and/or cutting of the sutures 28. As best shown in FIG. 6, the inner shaft 304 can include one or more lumens 306 (four in the illustrated embodiment) to receive the sutures 28.

A first suture 28a can extend through one of the lumens 306, through an eyelet 30 disposed on arm 24a of the second set of arms 20 and back through a respective lumen 306 to form a first suture loop. Similarly, a second suture 28b can extend through a respective lumen 306, through an eyelet 30 disposed on arm 24b of the second set of arms 20 and back through another lumen 306 to form a second suture loop. The proximal end portions of the sutures 28a, 28b may be held at a proximal end of the delivery apparatus by a retaining mechanism, (e.g., a stopcock (not shown) can be used as a retaining mechanism) and can be loosened and/or cut during or after implantation of the septal port device 10. In alternative embodiments, the inner shaft 304 can have greater or fewer number of lumens for the sutures. For example, the inner shaft 304 can include a single lumen 306 through which the sutures extend, or two lumens, one for each suture 28a, 28b.

Prior to implantation, the closure device 10 can be radially compressed to the delivery configuration and loaded into the distal end portion of the sheath 302. FIG. 3 shows the sutures threaded through the eyelets 30 and tensioned to fold the arms 20 toward each other. The sheath 302 can be advanced distally and/or the inner shaft 304 can be retracted proximally to draw the closure device 10 into the sheath 302 (FIG. 4). Once loaded in the sheath 302, the delivery apparatus 300 can be advanced percutaneously through the patient's vasculature to the right atrium of the heart in a trans-septal, antegrade approach for implanting the closure device 10 in the septum 100. In one approach, the delivery apparatus 300 can be advanced through a femoral vein, the inferior vena cava, and into the right atrium. In another approach, the delivery apparatus 300 can be advanced through a vein of the upper torso (e.g., a jugular vein), the superior vena cava, and into the right atrium.

Once in the right atrium, the delivery apparatus 300 can be advanced through the septum 100 to position a distal end portion of the sheath 302 in the left atrium. As shown in FIG. 5, the sheath 302 can then be retracted proximally to deploy the first set of arms 18 of the closure device 10, allowing the first set of arms 18 to radially expand within the left atrium. The entire delivery apparatus 300 can then be retracted and/or otherwise positioned to bring the expanded first end portion 26 against the septum 100 within the left atrium, as shown in FIG. 5. Thereafter, as shown in FIG. 6, the sutures 28 can be loosened and/or cut to deploy the central portion 14 and the second set of arms 20, allowing the second set of arms 20 to radially expand against the septum 100 in the right atrium. Once the sutures 28 are cut, the delivery apparatus 300 can be retracted, leaving the closure device 10 implanted in the orifice 10 in the septum 100. The clamping force of the first set of arms 18 and the second set of arms 20 against the opposing sides of the septum 100 can retain the closure device 10 in the orifice 102 (see FIGS. 19 and 20).

In particular embodiments, the central portion 14 is selected to have a diameter larger than the orifice 102 such that the central portion can reside entirely on one side of the orifice 102, except where the transition regions between the central portion 14 and the arms 16 extend through the orifice 102. For example, in the implementation shown in FIGS. 4-6, the central portion 14 of the frame 12 is deployed against the septum 100 within the left atrium. In another implementation, the central portion 14 can be deployed against the septum 100 within the right atrium. In other embodiments, the central portion 14 can have a diameter that is about the same or slightly less than the diameter of the orifice 102, in which case the central portion 14 can reside mostly within the orifice 102 when the closure device is fully deployed.

The closure device 10 is repositionable and recapturable at all times during delivery prior to cutting or removing the sutures 28 by tightening the sutures 28 as necessary and retracting the inner shaft 304 into the outer sheath 302 to re-collapse and draw the closure device back into the sheath. Also, the delivery apparatus 300 can be configured to rotate the closure device 10 through 360 degrees relative to the longitudinal axis B of the delivery apparatus during the implantation procedure to position the arms 16 at desired locations to accommodate variations in patient anatomy. For example, during the implantation procedure, it may be desirable to rotate the closure device 10 to avoid contact between the arms 16 and the aortic valve and/or the mitral valve. In the illustrated embodiment, the sutures 28, when held under tension, can retain the proximal arms 20 against the distal end of the inner shaft 304 such that rotating the inner shaft causes corresponding rotation of the closure device 10 (in the directions indicated by arrow 310 in FIG. 5).

The right atrium provides a relatively small working space between the septum 100 and the opposing wall of the right atrium in which the distal end portion of a delivery catheter can be manipulated for proper placement of a closure device within the septum. Due to the limited working space, as shown in FIGS. 4-6, the distal end portion of the delivery apparatus 300 can be advanced and retracted through the septum 100 at an acute angle 54 defined between the central longitudinal axis B of the delivery apparatus 300 and the septum 100. The angle 54 can be, for example, 90 degrees or less, 70 degrees or less, 50 degrees or less, 30 degrees or less, or 20 degrees of less.

Advantageously, the closure device 10 can be pivoted or angled relative to the longitudinal axis B of the delivery apparatus 300 to help position the closure device relative to the septum 100 while the delivery apparatus 300 is at an acute angle relative to the septum. As shown in FIG. 5, for example, the sutures 28 allow the closure device 10 to pivot relative to the distal end of the inner shaft 302 (which extends at an acute angle relative to the septum 100) to position the closure device with its central axis A normal to the septum 100 and non-parallel to the longitudinal axis B of the delivery apparatus. As such, even though the delivery apparatus 300 is at an acute angle, the distal arms 18 can be placed flush against the septum 100, which facilitates the final placement of the closure device as the delivery apparatus is retracted and the proximal arms 20 are deployed within the right atrium. In alternative embodiments, the delivery apparatus 300 can be advanced and retracted through the septum 100 at a normal angle with respect to the septum 100.

The delivery apparatus 300 greatly facilitates implantation of the closure device 10. In some embodiments, for example, following placement of a guidewire in the patient's body, the closure device 10 can be inserted into the patient's body, implanted in the septum, and released from the delivery apparatus in less than three minutes.

FIG. 19 shows the septum 100 and the closure device 10 just after implantation, also referred to the first stage of implantation, specifically when the occluding member 38 is not yet degraded and covers the septal defect 102. FIG. 20 shows the septum 100 and closure device 10 after the occluding member 38 has degraded and regrowth of the atrial tissue has occurred, also referred to as the second stage of implantation. During either the first stage or the second stage of implantation, the septal defect 102 can be accessed for reentry through the defect by any means known to those skilled in the art with the benefit of the present disclosure.

In some embodiments, the occluding member 38 and/or regrown tissue can be punctured with a medical instrument (e.g., a catheter) if access through the septum 100 is needed in a subsequent procedure. If the medical instrument has a relatively small diameter, such as used for treating arrhythmias, the hole formed in the occluding member 38 and/or regrown tissue may be small enough to sufficiently inhibit blood flow between the left and right atriums without further intervention. If the medical instrument has a relatively large diameter, such as a delivery apparatus for implanting prosthetic valve, and leaves a relatively larger opening in the occluding member 38 and/or regrown tissue, another closure device can be implanted within the first device 10 to block blood flow between the right and left atriums.

Additionally, the central portion 14 of the frame 12 can be expandable to accommodate entry of a medical instrument that has a larger diameter than the central portion 14 at rest. For example, the central portion 14 shown in FIGS. 1 and 2 is configured to be expandable as it is not a closed loop and is instead formed with circumferentially spaced gaps 52 at the plurality of arms, thereby allowing deformation and/or expansion. Additionally and/or alternatively, the central portion of the frame can be configured to be expandable if formed from a loop of a wire that can deform as needed to accommodate an instrument, such as the embodiments shown in FIGS. 13-16, which are further described below.

Various types of medical instruments can be passed through the closure device to access the left side of the heart. The medical instrument can be, for example, a delivery apparatus for delivering and implanting a prosthetic heart valve in the native mitral valve or the native aortic valve. In alternative embodiments, the delivery apparatus can be used to deliver and implant various other prosthetic devices in the left atrium, mitral valve, left ventricle, and/or the aortic valve, including, for example, annuloplasty rings, closure devices for the left atrial appendage, sealing devices or reshaping devices for resetting or reshaping portions of the heart. In other embodiments, other percutaneous medical instruments can be advanced through the port device 20 for performing a procedure on the left side of the heart, such as atrial fibrillation therapy.

FIGS. 7 and 8 show a septal closure device 200, according to another embodiment. The closure device 200 can include a frame 12, an occluding member 42 supported on the frame 12, and a backing ring 44. The occluding member 42 and the backing ring 44 can be aligned with the central axis with the central portion 14 of the frame disposed in between, as shown in FIG. 7, and then bonded together, as shown in FIG. 8, via heat staking, ultrasonic welding, sutures or other means of attachment to the frame 12 known to those skill in the art with the benefit of the present disclosure.

FIG. 10 shows a septal closure device 400, according to another embodiment. The closure device 400 can include a frame 402 supporting an occluding member 404. FIG. 11 shows the bare frame 402 with the occluding member 404 removed for purposes of illustration. The frame 402 in the illustrated configuration includes a generally square central portion 406 and a plurality of generally elliptical arms 408 (four in the illustrated embodiment) extending radially from the central portion 406. The central portion 406 can include convex corner portions 410 between the locations where the ends of each arm 408 connects to the central portion 406. As shown in FIG. 11, the corner portions 410 can facilitate expansion of the central portion 406 into a substantially circular shape when a medical device is passed through the central portion (as indicated by the dashed circle 412 in FIG. 11). The occluding member 404 can cover the entire central portion 406 except at the corners 410 to facilitate expansion of the central portion.

FIG. 12 shows a septal closure device 400', according to another embodiment, which comprises a frame 402 and an occluding member 420 supported on the frame 402. In this embodiment, the closure device 400' is similar to the closure device 400, except that the occluding member 420 covers the entire central portion 406.

FIGS. 13-16 show alternative frames that can be incorporated into a septal closure device. FIG. 13 shows a frame 450 similar in overall shape to the frame 402 and comprises a generally square central portion 452 and a plurality of generally elliptical arms 454 (four in the illustrated embodiment) extending radially from the central portion 452. The frame 450 can be formed by a bending a single metal wire into multiple loops defining the central portion and each of the arms. The ends of metal wire can be un-joined or can be joined via crimping, welding, braise alloy, sutures, sleeves (bioresorbable or non-bioresorbable) or other means of joining know to those of skill of the art with the benefit of the present disclosure. In some embodiments, an end 456 of the wire loop forming the central portion 452 can be left un-joined to the adjacent portion of the frame to facilitate expansion of the central portion if a medical instrument is passed through the closure device during a subsequent procedure.

The wire used for forming the frame 452 can be made of super-elastic material (e.g., Nitinol) and/or can be shaped via heat setting. In other embodiments, the wire can be made of other biocompatible materials, including any of various polymers or metals (e.g., stainless steel) and can be shaped to self-expand from a compressed, delivery configuration to an expanded, deployed configuration when deployed from a delivery sheath.

FIG. 14 shows a frame 460 comprising a generally circular central portion 462 and a plurality of generally elliptical arms 464 (six in the illustrated embodiment) extending radially from the central portion 462. FIG. 15 shows a frame 470 comprising a generally circular central portion 472 and a plurality of generally diamond-shaped arms 474 (four in the illustrated embodiment) extending radially from the central portion 472.

FIG. 16 shows a frame 480 comprising a generally circular central portion 482 and a plurality of generally circular arms 484 (four in the illustrated embodiment) extending radially from the central portion 482. The arms 484 can be slightly bent relative to the central portion 482 to aid in anchoring the frame to a septum. For example, the arms 484 can include a first set of opposing arms 484a bent in a first direction relative to the central portion 482 and a second set of opposing arms 484b bent in a second, opposing direction relative to the central portion 482. The first set of arms 484a can be biased toward the second set of arms 484b such that when the frame is implanted with arms 484a on one side of the septum and arms 484b on the other side of the septum, the arms are urged against opposing sides of the septum.

The frames shown in FIGS. 13-16 are formed from bending a single metal wire into the shapes illustrated in the figures. In other embodiments, each of arms and the central portion can be formed from separate wires and subsequently secured to each other, such as by welding, an adhesive, sutures, or other techniques or mechanisms. In still other embodiments, any of the frames described above can be formed by machining (e.g., laser cutting) the frame from a flat piece of metal or other suitable material.

FIGS. 17-18 show a closure device 500, according to another embodiment. The closure device 500 can include a frame 502 and an occluding member 504 supported on the frame. The frame 502 can have any of various the frame configurations disclosed herein. The occluding member 504 can include a plurality of overlapping "leaflets" or flaps 506a, 506b, 506c that are arranged relative to each other to maintain a closed position against a blood pressure gradient between the right atrium and the left atrium but can be opened by the force of a catheter or other medical instrument to permit passage of the medical instrument through the lumen 26 of the closure device 500. The flaps primarily block the flow of blood from the left atrium to the right atrium due to the typically higher LAP, but can also block the flow of blood from the right atrium to the left atrium if the RAP exceeds the LAP. The plurality of overlapping flaps 506a, 506b, 506c can also be described as forming an expandable hole 508, the hole 508 having a closed configuration to block at least the flow of blood from the left atrium to the right atrium through the hole 508 and an expanded configuration to permit a medical instrument inserted in the right atrium to pass through the lumen and the valve member and into the left atrium.

Each flap 506a-506c can comprise an angular wedge-shaped or pie-shaped segment comprising an outer peripheral edge portion and radially extending side edge portions. The flaps 506a-506c can be secured to the central portion 14 of the frame 502 using suitable techniques or mechanisms known to those skilled in the art with the benefit of the present disclosure. For example, outer peripheral edges of the flaps 506a-506c can be secured to the frame 502, such as with sutures, an adhesive, and/or welding. Each of the radially extending edge portions of a flap can overlap an adjacent edge portion of an adjacent flap. The radially extending edge portions of the flaps can be unattached to the frame 502 and to each other. In some embodiments, the radially extending side edge portions can be secured to each other or to the frame 502 proximate the outer peripheral edge portions so long as the flaps can be opened by the force of a medical instrument inserted through the lumen 26 of the device 500.

Although three flaps 506a-506c are shown in the illustrated embodiment, a greater or fewer number of flaps can be used in alternative embodiments. Also, the flaps 506a-506c can be equally sized and shaped, while in other embodiments the flaps can comprise different sized angular segments. In particular embodiments, for example, each flap comprises an angular segment that has an angle greater than 90 degrees between the radially extending sides, such as about 100 to 120 degrees. In other embodiments, each flap 506a-506c can subtend a different angle between the radially extending sides.

The flaps 506a-506c can be formed from any of various suitable materials disclosed herein, including natural tissue or synthetic materials, such as any of various electro-spun polymers, woven (e.g., fabric) or non-woven materials made from any of various polymeric materials. Some examples of natural tissue include, for example, bovine, porcine, or equine pericardial tissue or pericardial tissue from other animals. Some examples suitable polymeric materials include, for example, polyurethane or polyester. In one specific example, the flaps can comprise polyethylene terephthalate (PET) fabric.

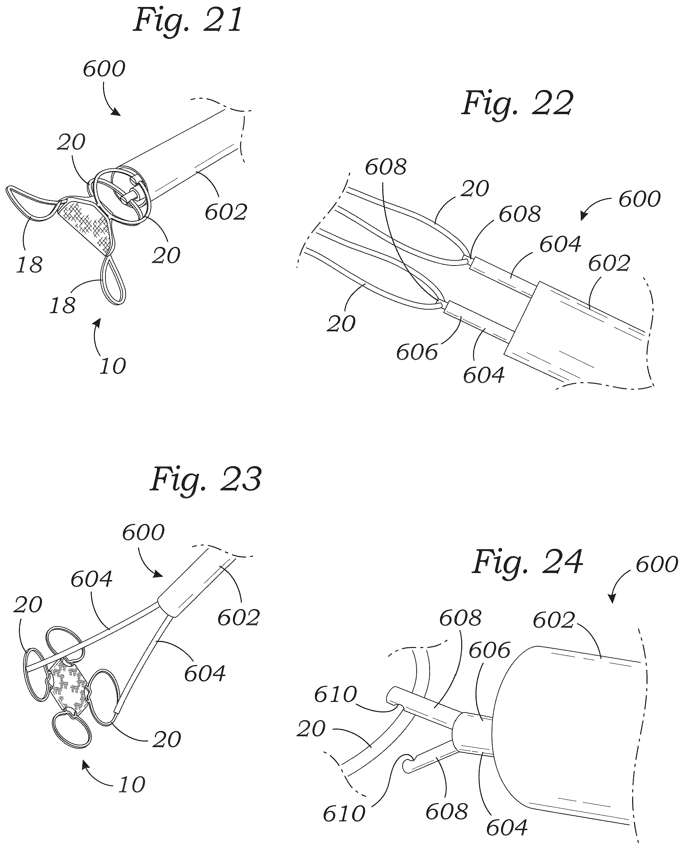

FIGS. 21-24 show another embodiment of a delivery apparatus, indicated generally at 600, that can be used to implant any of the closure devices disclosed herein (e.g., a closure device 10). The delivery apparatus 600 can include a sheath 602 and retaining arm assemblies 604 extending through a lumen of the sheath 602. The retaining arm assemblies 604 can be releasably attached to the proximal arms 20 of the closure device 10. As best shown in FIG. 24, in the illustrated embodiment, each retaining arm assembly 604 can include an outer sheath 606 that extends over a pair of pinching members or claws 608.

Each pair of claws 608 can be configured to clamp a proximal arm 20 of the closure device 10 between the claws. The claws 608 can be normally biased away from each other to an open position using a spring (not shown) or other type of biasing mechanism and can be held in a closed position by sliding the sheath 606 over the claws (FIG. 22). Each claw 608 can be formed with a notch or groove 610 on a distal inner surface thereof to receive a portion of an arm 20 of the closure device when the claws are held in the their closed position.

FIG. 21 shows the closure device 10 in the partially deployed configuration with the closure device 10 advanced from the distal end of the sheath 602 and the retaining arm assemblies 604 substantially within the sheath 602 and connected to the proximal arms 20 of the closure device 10. Each sheath 606 extends over a respective pair of claws 608 to hold them in a closed position gripping a respective arm 20 of the closure device 10. FIG. 22 shows the sheath 602 slightly retracted, exposing the distal end portions of the retaining arm assemblies 604. In the partially deployed state of the closure device shown in FIGS. 21-22, the delivery apparatus can be manipulated (advanced, retracted, or rotated) to position the distal arms 18 of the closure device at desired positions within the left atrium.

FIG. 23 shows the sheath 602 further retracted relative to the retaining arm assemblies 604 a distance sufficient to allow the retaining arm assemblies splay apart from each other under the resiliency of the frame 12. The retaining arm assemblies 604 have sufficient flexibility to allow the proximal arms 20 of the closure device 10 to fully expand upon further retraction of the sheath 602. FIG. 24 is a close up view showing the sheath 606 of a retaining arm assembly retracted relative to a pair of respective claws 608, which allows the claws to move to their open position, thereby releasing the respective arm 20 of the closure device. Each retaining arm assembly is actuated in this manner to release the claws 608 from the proximal arms 20 of the closure device, allowing the proximal arms to engage the septum 100 in the right atrium.

In alternative embodiments, the retaining arm assemblies 604 can be releasably attached to the proximal arms 20 of the closure device via sutures or other suitable attachment structures, in lieu of or in addition to the claws 608.

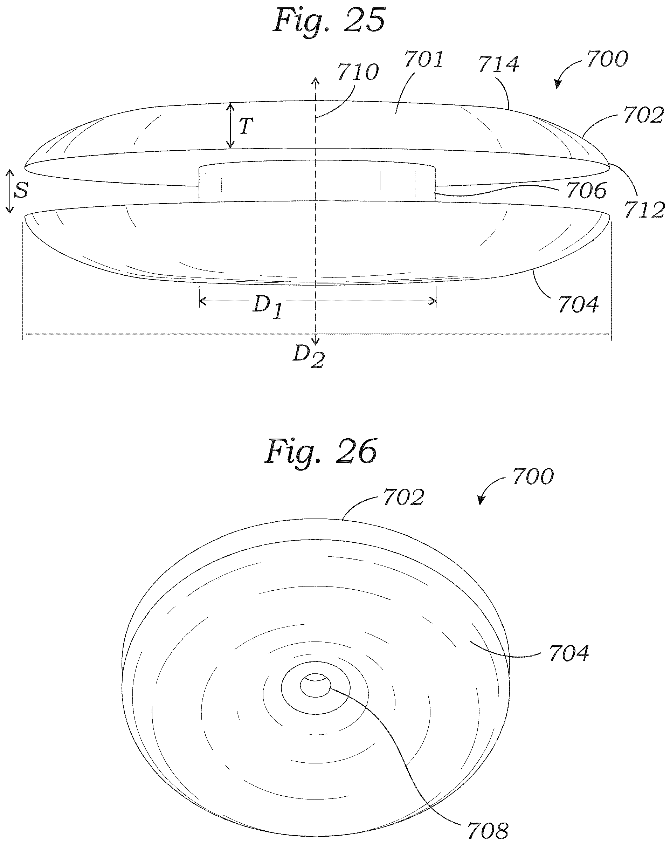

FIGS. 25-26 show a septal closure device 700, according to another embodiment. The closure device 700 can generally comprise an expandable body 701 comprising a first end portion 702, a second end portion 704 and a central portion 706 between the first and second end portions 702, 704. In its deployed state, the first and second end portions 702, 704 can extend radially outwardly from opposite ends of the central portion 706, thereby forming opposing flange portions. The first and second end portions 702, 704 can extend perpendicularly or substantially perpendicularly to a central axis 710 of the device 700 (the central axis extending from the first end to the second end of the device 700) and can compress or pinch the atrial septum 100 between the end portions 702, 704 when the device 700 is implanted in the atrial septum 100. Additionally and/or alternatively, the central portion 706 can include an outer diameter that is greater than the diameter of the orifice 102 in which it is to be implanted. Additionally and/or alternatively, the device 700 may include one or more anchoring elements that aid in the retention of the device 700 in the septal orifice 102.

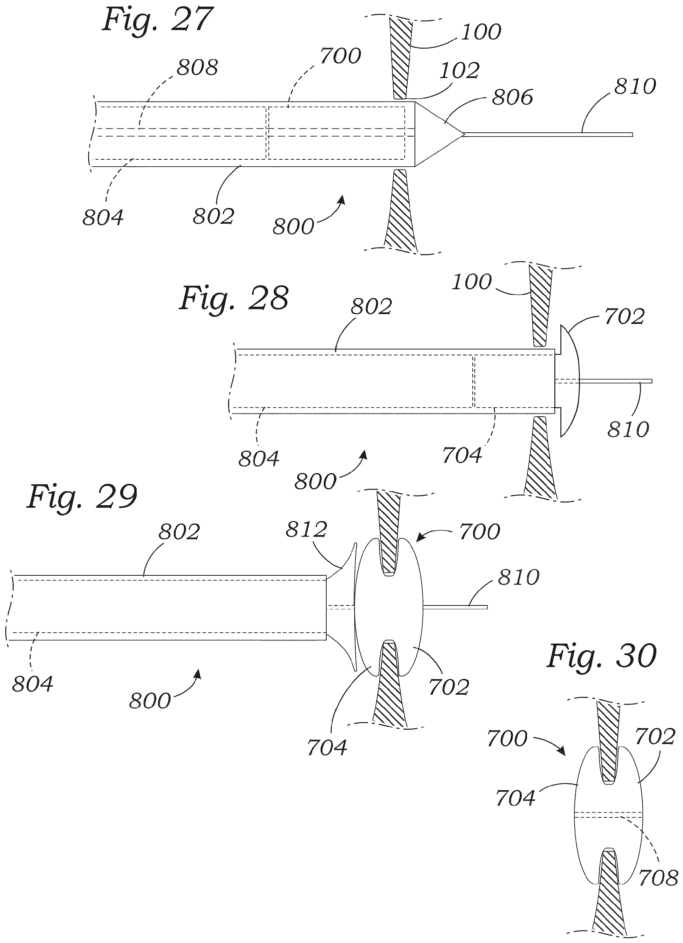

The device 700 can be radially compressed or constricted to a delivery configuration for delivery to the heart on a delivery apparatus 800, as shown in FIG. 27. In the delivery configuration, the device 700 can be placed and retained in a generally cylindrical or tubular configuration in which the central portion 706 is radially compressed and the first and second end portions 702, 704 are also radially compressed and/or folded toward the central axis 710 of the device 700.

The device 700 can be self-expandable so that the device 700 self-expands from the delivery configuration (FIG. 27) to the deployed configuration (FIGS. 25 and 30) when released or deployed from a delivery apparatus. In particular embodiments, the body 701 is made of a biocompatible foam, such as polyurethane, PET, silicone, or polyethylene foam, and desirably contains no metal components. In some embodiments, the foam material can be a bioresorbable material, such as a foam material formed from any of the bioresorbable polymers previously described above. The porous nature of the foam material can promote tissue ingrowth, thereby closing the septal defect 102 with newly grown tissue. The compressible and resilient nature of the foam material allows the device to be radially compressed to a small diameter for insertion into a sheath of a delivery apparatus and causes the device to self-expand to its deployed configuration once deployed from the sheath. In addition, the foam material can be impregnated with radiopaque additives for fluoroscopic visualization, for example, barium salts (e.g., barium sulfate) or other additives known to those skilled in the art with the benefit of the present disclosure.

In particular embodiments, the entire body 701 is formed as a unitary structure without any seams or connections between the central portion 706 and the first and second end portions 702, 704. In other words, the central portion 706 and the first and second end portions 702, 704 can be integrally formed with each other, such as by molding the entire device from a curable material, or by shaping or forming the device from a blank of material (e.g., three-dimensional printing). In alternative embodiments, one or more of the central portion and the first and second end portions can be separately formed and subsequently connected to each other, such as by welding or an adhesive.

Some embodiments of the device 700 can include a lumen 708 extending from the first end portion 702 to the second end portion 704. As shown, the central portion 706 can be cylindrical in shape, and each of the first and second end portions 702, 704 can be disc-shaped having a maximum outer diameter at a first end 712 adjacent the central portion and tapering to a smaller diameter at a second end 714 defining one of the terminal ends of the device 700. In another embodiment, the first and second end portions 702, 704 can be cylindrical in shape. In still other embodiments, each end portion can have a maximum diameter at a location between the ends 712, 714 and can taper to smaller diameters at the ends 712, 714, similar to a donut shape.

As shown in FIG. 25, the device 700 in the deployed configuration can include an inner diameter D1 and an outer diameter D2. The inner diameter D1 can be defined by the central portion and can be slightly less to slightly greater than that the diameter of the orifice in the septum. The outer diameter D2 can be defined by the first and second portions.

In certain embodiments, the inner diameter D1 can be between about 5 mm and 15 mm, and more specifically, between about 6 mm and 12 mm, with 10 mm being a specific example. The outer diameter D2 can be between about 12 mm and 36 mm, and more specifically, between about 20 mm and 30 mm, with 20 mm being a specific example. The spacing S between the first and second end portions 702, 704 can be between about .5 mm and 10 mm, and more specifically, between about 1 mm and 8 mm, with 1 mm being a specific example. The thickness T of the first or second end portion 702, 704 can be between about 1 mm and 10 mm, and more specifically, between about 2 mm and 8 mm, with 3 mm being a specific example. These dimensions can be varied as needed for particular applications of the device.

In some embodiments, the device 700 can comprise first and second end portions 702, 704 separated by a slit without a central portion 706. In such embodiments, the spacing S between the end portions 702, 704 can be zero, although the resiliency of the end portions allows the tissue of the septum to be inserted into the slit between the end portions.

The device 700 can be configured to block the flow of blood between the right and left atria through the device 700 but permit passage of a medical device through the central portion 706 of the device, before or after degradation of the device 700 and regrowth of tissue in the defect 102. The lumen 708 can be sized such that a medical device (e.g., a delivery catheter) can be easily inserted through the lumen 708 and expand the lumen if the diameter of the medical device is greater than the diameter of the lumen in its non-deformed state. Over time, the lumen 708 can become completely closed or sealed through tissue ingrowth. In some embodiments, instead of an open lumen 708, the device can have an axially extending slit extending from the first end portion to the second end portion completely through the device. The axially extending slit can be completely closed in its non-deformed state to limit residual shunting immediately upon implantation, but allows a medical device to be inserted through the slit.

FIGS. 27-30 illustrate one example of delivering and implanting the closure device 700 using an exemplary delivery apparatus 800. The delivery apparatus 800 can generally comprise a sheath 802, an inner shaft or pusher member 804 extending co-axially through the outer sheath 802, and a nose cone 806. The nose cone can be mounted on an innermost nose cone shaft 808 that extends co-axially through the pusher member 804. The nose cone 806 and the nose cone shaft 808 can include a lumen sized to allow the delivery apparatus 800 to be advanced over a guidewire 810. The proximal ends of the sheath 802, the pusher member 804, and the nose cone shaft 808 can be coupled to a handle (not shown) having appropriate actuators (e.g., knobs) to effect relative longitudinal and/or rotational movement of these components relative to each other.

Prior to implantation, the closure device 700 can be radially compressed to the delivery configuration and loaded into the distal end portion of the sheath 802. The distal end portion of the nose cone shaft 808 can extend through the lumen 708 of the closure device, as depicted in FIG. 27. The delivery apparatus 800 can be advanced percutaneously through the patient's vasculature to the right atrium of the heart in a trans-septal, antegrade approach for implanting the closure device 700 in the septum 100. In one approach, the delivery apparatus 800 can be advanced through a femoral vein, the inferior vena cava, and into the right atrium. In another approach, the delivery apparatus can be advanced through a vein of the upper torso (e.g., a jugular vein), the superior vena cava, and into the right atrium.

Once in the right atrium, the delivery apparatus 800 can be advanced through the septum 100 to position the nose cone 806 and a distal end portion of the sheath 802 in the left atrium, as shown in FIG. 27. If there is an existing orifice 102 in the septum 100 (e.g., from a congenital defect), the delivery apparatus 800 can be advanced through the orifice 102. If the closure device 700 is being used to provide an access port in a healthy septum to perform a procedure on the left side of the heart, the guidewire 810 and/or the nose cone 806 can be used to puncture the septum 100 and create an orifice 102.

As shown in FIG. 28, the sheath 802 can then be retracted proximally to deploy the first end portion 702 of the closure device, allowing the first end portion 702 to radially expand. Alternatively, the first end portion 702 can be deployed by advancing the closure device distally relative to the sheath 802 and/or retracting the sheath while advancing the closure device distally. The nose cone 606 (not shown in FIG. 28 for purposes of illustration) can be advanced distally away from the distal end of the sheath to permit deployment of the first end portion 702. The entire delivery apparatus 800 can then be retracted slightly to bring the expanded first end portion 702 against the septum 100 within the left atrium.

Thereafter, as shown in FIG. 29, the sheath 802 can be further retracted to deploy the central portion 706 and the second end portion 704, allowing the second portion 704 to radially expand against the septum 100 in the right atrium, and allowing the central portion to radially expand within the orifice 102, leaving the closure device 700 implanted in the orifice 102 in the septum (alternatively, the central portion and the second end portion can be deployed by advancing the closure device distally relative to the sheath and/or retracting the sheath while advancing the closure device distally). The pusher member 804 can have a radially expandable distal end portion 812 that expands upon retraction of the sheath 802 to help push the expanded second end portion 704 against the septum. The expandable distal end portion 812 can comprise, for example, an expandable tubular member, or alternatively, a plurality of retaining arms that splay apart from each other when advanced from the sheath (similar to retaining arm assemblies 604 of FIG. 23). The distal end portion 812 can be made of a radiopaque material or can include radiopaque components that renders the distal end portion 812 visible under fluoroscopy so as to aid in positioning the second end portion 704.

The clamping force and/or friction of the first end portion 702 and the second end portion 704 against the opposing sides of the septum can retain the closure device 700 in the orifice. Additionally and/or alternatively, the radial outwardly directed force and/or friction of the central portion 706 against the orifice 102 can assist in retaining the device 700 in the orifice 102. The guidewire 810 can be temporarily left in place if another medical instrument is to be used to access the left side of the heart in a subsequent procedure.

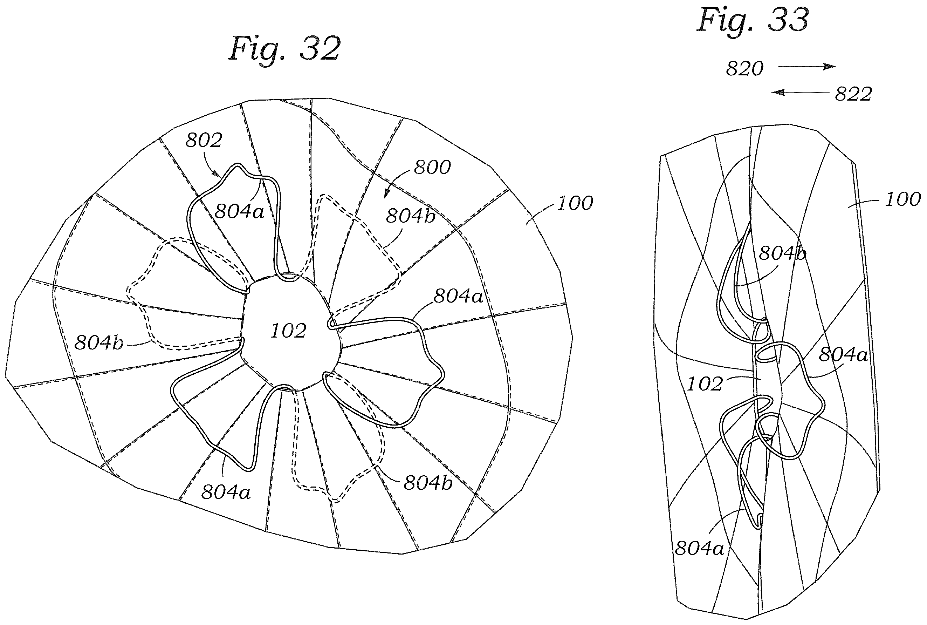

FIGS. 31A, 31B, 32 and 33 show an exemplary septal closure device 800, according to another embodiment, implanted within an orifice 102 of a septum 100. The septal closure device 800 comprises a frame 802. In particular embodiments, the frame 802 can comprise a closed loop wire-form that can be formed from a single wire that is bent into the shape shown in FIGS. 31A-31B. In other embodiments, the frame 802 can be cut (e.g., laser cut) from a blank (e.g., a flat or tubular piece of material) and then shape set in the form shown. The frame 802 can be formed from any of the materials described above in connection with the frames of the previously described embodiments. The device 800 can include an occluding member as previously described.

The frame 802 can have a first set of anchoring arms 804a and a second set of anchoring arms 804b. Each of the arms 804a in the illustrated embodiment can have two side portions 806a, two upper portions 808a extending toward each other from respective radial outer ends of the side portions 806a, and a tip 810a formed between the upper portions 808a. In the illustrated example of FIG. 31A, the side portions 806a are bent slightly away from each other as they extend radially outwardly from their radial inner ends. In other examples, the side portions 806a can be substantially parallel or can be bent towards each other moving in a direction outwardly from their radial inner ends. The upper portions 808a can be substantially perpendicular to the side portions 806a. The tip 810a can be U-shaped, as shown in FIG. 31A. In some examples, the tip 810a is not present in the arms 804a and the upper portions 808a comprise a straight section extending between the outer ends of the side portions 806a. Each of the arms 804b can be constructed in a similar manner to the arms 804a and can comprise two side portions 806b, two upper portions 808b, and a tip 810b.

In the illustrated example, there are three anchoring arms 804a and three anchoring arms 804b. In other examples, there can be any number of anchoring arms 804a and 804b. Each anchoring arm 804a can be connected to two adjacent anchoring arms 804b by two U-shaped connecting portions 812. Each U-shaped portion 812 extends from a side portion 806a of the arm 804a to an adjacent side portion 806b of an adjacent arm 804b. As best shown in FIG. 31B, each U-shaped portion 812 extends generally axially as it extends from a side portion 806a of an arm 804a to a side portion 806b of an arm 804b. Thus, the shape of the U-shaped portion 812 spaces apart the radial inner ends of the side portion 806a and the side portion 806b in the axial direction (a direction parallel to a central axis A extending through the center of the frame).

As can best be seen in FIG. 31B (which shows the device 800 implanted in a septum 100), the arms 804a are bent or curved in a first direction 820 toward the arms 804b and the arms 804b are bent or curved in a second direction 822 toward the arms 804a. Accordingly, when the closure device 800 is implanted in a septum 100 having an orifice 102, the first set of anchoring arms 804a press against a first side of the septum and the second set of anchoring arms 804b press against a second side of the septum to hold the closure device.

The frame 802 can be made from a shape-memory material such as Nitinol, and can be shape set in the shape shown in FIGS. 31A, 31B. As such, during an implantation procedure, discussed below, the frame 802 naturally assumes the shape of FIGS. 31A, 31B. The delivery apparatus and technique described above for the closure device 10 can be used to implant the closure device 800. Similar to the previously described embodiments, the central portion of the frame defined by the U-shaped portions 812 can expand radially to accommodate a larger medical device that is inserted through the closure device 800 in a subsequent procedure.

In alternative embodiments, any of the frames described above in connection with FIGS. 1-24 (e.g., any of frames 12, 12', 402, 450, 460, 470, 480, 500, 802), when provided without a blood-occluding member, can be used to maintain the patency of an orifice in tissue and therefore can be used a shunt to permit fluid (e.g., blood) to flow through the orifice. For example, a frame (e.g., any of frames 12, 12', 402, 450, 460, 470, 480, 500, 802) can be implanted in an orifice in the atrial septum to allow blood to flow from the left atrium into the right atrium to reduce pressure in the left atrium, which can help treat pulmonary hypertension. Eliminating the blood-occluding member inhibits healing of the orifice and instead the frame functions to maintain an opening between the atria sufficient to reduce blood pressure in the left atrium.

In particular embodiments, a method of treating pulmonary hypertension comprises forming an orifice in the atrial septum (e.g., a 7-9 mm orifice) using a needle inserted through the vasculature of a patient (e.g., through the inferior or superior vena cava) and into the right atrium of the heart. The end of the needle is used to puncture the atrial septum and form the orifice. Thereafter, a shunt comprising any of frames 12, 12', 402, 450, 460, 470, 480, 500 (or any of the modifications of these frames described above) can be implanted in the orifice, such as using the delivery apparatus of FIGS. 3-6 or the delivery apparatus of FIGS. 21-24 and the method described above corresponding to the delivery apparatus.

### General Considerations

For purposes of this description, certain aspects, advantages, and novel features of the embodiments of this disclosure are described herein. Features, integers, characteristics, compounds, chemical moieties or groups described in conjunction with a particular aspect, embodiment or example of the disclosure are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The disclosure is not restricted to the details of any foregoing embodiments. The disclosure extends to any novel one, or any novel combination, of the features disclosed in this specification (including any accompanying claims, abstract and drawings), or to any novel one, or any novel combination, of the steps of any method or process so disclosed.

Although the operations of some of the disclosed methods are described in a particular, sequential order for convenient presentation, it should be understood that this manner of description encompasses rearrangement, unless a particular ordering is required by specific language. For example, operations described sequentially may in some cases be rearranged or performed concurrently. Moreover, for the sake of simplicity, the attached figures may not show the various ways in which the disclosed methods can be used in conjunction with other methods.

As used herein, the terms "a", "an", and "at least one" encompass one or more of the specified element. That is, if two of a particular element are present, one of these elements is also present and thus "an" element is present. The terms "a plurality of" and "plural" mean two or more of the specified element.

As used herein, the term "and/or" used between the last two of a list of elements means any one or more of the listed elements. For example, the phrase "A, B, and/or C" means "A", "B,", "C", "A and B", "A and C", "B and C", or "A, B, and C."

As used herein, the term "coupled" generally means physically coupled or linked and does not exclude the presence of intermediate elements between the coupled items absent specific contrary language.

In view of the many possible embodiments to which the principles of the disclosed technology may be applied, it should be recognized that the illustrated embodiments are only preferred examples of the disclosure and should not be taken as limiting the scope of the disclosure. Rather, the scope of the disclosure is defined by the following claims. We therefore claim as our disclosure all that comes within the scope and spirit of these claims.

The invention further comprises the following embodiments:
1. A closure device for implantation in an orifice formed in an organ of a patient's body, comprising:
   an expandable frame comprising a central portion defining a lumen, the lumen defining a central axis, the frame further comprising a plurality of arms extending radially outward from the central portion, the frame being configured to expand and contract between a compressed configuration for delivery through the patient's vasculature and an expanded configuration in which the arms extend radially outwardly from the central portion, the arms being connected to the central portion at angularly spaced locations on the central portion that intersect a common plane perpendicular to the central axis; and
   a blood occluding member supported on the frame and positioned to block at least the flow of blood from one side of the orifice to the other side of the orifice through the lumen of the frame.
2. The device of embodiment 1, wherein the plurality of arms comprises a first set of two or more arms that can be positioned on one side of the orifice and a second set of two or more arms that can be positioned on the other side of the orifice.
3. The device of embodiment 2, wherein there are a total of four arms, including exactly two arms in the first set and exactly two arms in the second set.
4. The device of embodiment 3, wherein the arms of the first set extend from opposing sides of the central portion and the arms of the second set extend from opposing sides of the central portion.
5. The device of any preceding embodiment, wherein the arms do not overlap with each other when in the expanded configuration.
6. The device of any preceding embodiment, wherein each of the arms has a first portion where it is connected to the central portion and a relatively wider, second portion spaced from the central portion.
7. The device of any preceding embodiment, wherein the occluding member comprises a bioresorbable material.
8. The device of any preceding embodiment, wherein the occluding member comprises an electro-spun polymer.
9. The device of any of embodiments 1-7, wherein the occluding member comprises a fabric.
10. The device of any of embodiments 1-7, wherein the occluding member comprises a foam.
11. The device of any preceding embodiment, wherein the central portion is further expandable from the expanded configuration when a medical instrument is inserted through the lumen.
12. The device of any preceding embodiment, wherein the arms are coplanar with the central portion when the frame is in the expanded configuration.
13. The device of any preceding embodiment, wherein the central portion of the frame comprises a circumference and each arm is connected to the central portion at spaced apart locations on the circumference.
14. The device of any preceding embodiment, wherein the central portion of the frame comprises a central loop and each arm comprises a respective loop connected to the central loop at spaced apart locations around the central loop.
15. The device of any preceding embodiment, wherein the blood occluding member is configured to be punctured by a medical instrument.
16. A method of making an implantable closure device, the method comprising:
   cutting a frame from a flat piece of metal, the frame having a central portion defining a lumen and a plurality of arms extending radially outward from the central portion, one or more of the arms being configured to be positioned against tissue on one side of an orifice in a patient's body and one or more of the arms being configured to be positioned against tissue on the opposite side of the orifice; and
   securing a blood-occluding member to the frame so as to at least partially cover the lumen.
17. The method of embodiment 16, wherein the act of cutting comprises laser cutting the frame from the flat piece of metal.
18. A septal closure device for implantation in the atrial septum of a patient's heart, comprising an expandable foam body comprising first and second opposing end portions, the body being configured to expand and contract between a compressed configuration for delivery through the patient's vasculature and an expanded configuration in which the first and second end portions are positioned on opposing sides of the atrial septum.
19. The closure device of embodiment 18, wherein the body further comprises a central portion and the first and second end portions extend radially outwardly from opposing ends of the central portion when the body is in the expanded configuration.
20. The closure device of any of embodiments 18-19, further comprising a radiopaque additive within the foam body.
21. The closure device of any of embodiments 18-20, wherein the foam body comprises a bioresorbable material.
22. A shunt for implantation in an orifice formed in an organ of a patient's body, comprising:
   an expandable frame comprising a central portion defining a lumen, the lumen defining a central axis, the frame further comprising a plurality of arms extending radially outward from the central portion, the frame being configured to expand and contract between a compressed configuration for delivery through the patient's vasculature and an expanded configuration in which the arms extend radially outwardly from the central portion, the arms being connected to the central portion at angularly spaced locations on the central portion that intersect a common plane perpendicular to the central axis.
23. An implantable medical device for implantation in an orifice formed in an organ of a patient's body, comprising:
   a metal frame comprising a plurality of loop shaped anchoring arms extending radially outwardly from a central axis of the frame and a plurality of connecting portions extending between and connecting adjacent anchoring arms, wherein the anchoring arms are angularly arrayed around the central axis and each anchoring arm comprises two radial inner ends with each radial inner end being connected to an adjacent radial inner end of an adjacent anchoring arm by one of the connecting portions;
   wherein the plurality of anchoring arms comprises a first set of anchoring arms that can be positioned on one side of the orifice and a second set of anchoring arms that can be positioned on the other side of the orifice;
   wherein the connecting portions define a central lumen of the frame having a first diameter;
   wherein the frame is configured such that when a medical instrument having a second diameter, greater than the first diameter, is inserted into the central lumen, the connecting portions are pushed radially outwardly to enlarge the lumen under the force of the medical instrument.
24. The implantable medical device of embodiment 23, further comprising a blood occluding member supported on the frame and positioned to block at least the flow of blood from one side of the orifice to the other side of the orifice through the lumen of the frame.
25. The implantable medical device of any of embodiments 23-24, wherein the plurality of anchoring arms and the connecting portions are formed from a single wire member.
26. The implantable medical device of any of embodiments 23-25, wherein the plurality of anchoring arms are substantially flat and co-planar with each other.
27. The implantable medical device of any of embodiments 23-25, wherein each of the plurality of anchoring arms curves away from and back toward an adjacent anchoring arm moving in a radial outward direction along the length of the anchoring arm.

## Claims

1. A closure device comprising:
a frame (12) comprising:
a plurality of anchoring arms (16) projecting radially outwardly from a central portion, defining a central lumen (26), the plurality of anchoring arms (16) being attached to the central portion (14) at angularly spaced locations and each comprising a radially-inner narrow portion (34) and a wider portion (36) radially outside of the narrow portion;
a plurality of connecting portions (15) extending between and connecting adjacent ones of the plurality of anchoring arms (16), the plurality of connecting portions (15) forming the central lumen (26); and
an occluding member (38) supported by the frame (12) comprising:
one or more sheets of material (38), the one or more sheets of material (38) at least partially covering the central lumen (26).

2. The closure device of claim 1, wherein the one or more sheets of material (38) comprises a plurality of overlapping flaps (506).

3. The closure device of claim 2, wherein the plurality of overlapping flaps (506a, 506b, 506c) are angular wedge-shaped or pie-shaped segments comprising radially extending side edge portions, wherein each of the radially extending edge portions of a flap (506a, 506b, 506c) can overlap an adjacent edge potion of an adjacent flap (506a, 506b, 506c).

4. The closure device of claim 1, wherein the plurality of anchoring arms (16) and the plurality of connecting portions (15) are formed of a single wire (12) that has first and second un-joined ends that facilitate expansion of the central lumen (26).

5. The closure device of claim 1, wherein each of the plurality of anchoring arms (16) includes an eyelet (30) disposed at a distal end thereof.

6. The closure device of claim 1, wherein the closure device has a total surface area that is less than 200 mm2 and a weight of less than 0.1 gram.

7. The closure device of claim 1, wherein the plurality of connecting portions are configured to deflect radially outwardly to enlarge the central lumen (26).

8. The closure device of claim 1, wherein the plurality of anchoring arms (16) comprises a first set (16) of two or more arms that can be positioned on one side of an orifice and a second set (18) of two or more arms that can be positioned on the other side of the orifice.

9. The closure device of claim 1, wherein there are a total of four anchoring arms (16), including exactly two arms in the first set (18) and exactly two arms in the second set (20).

10. The closure device of claim 1, wherein the anchoring arms (16) are coplanar with the central portion (14) when the frame is in the expanded configuration.

11. The closure device of claim 1, wherein the occluding member comprises a bioresorbable material.

12. A delivery apparatus comprising:
an outer sheath (302) including a distal end portion having disposed thereon a closure device (10) as defined in any one of claims 1 to 7, such that a first set (18) of the plurality of anchoring arms (16) are folded in a first direction and a second set (20) of the plurality of anchoring arms (16) are folded in the first direction; and
an inner shaft (304) releasably connected to at least one of the second set (20) of anchoring arms (16).

13. The delivery apparatus of claim 8, wherein the inner shaft (304) is releasably connected to the at least one of the second set (20) of anchoring arms (16) via one or more sutures (28) that run through an eyelet (30) of each of the at least one of the second set (20) of anchoring arms (16).

14. The delivery apparatus of claim 13, wherein the inner shaft (304) is releasably connected to the second set (20) of anchoring arms (16) via a retaining arm assembly (604).

15. The delivery apparatus of claim 13, wherein the inner shaft (304) is rotatable within the outer shaft (302) to cause rotation of the closure device.
